# EUROPEAN PATENT APPLICATION

(11) **EP 4 627 935 A1**
(43) Date of publication of application: **08.10.2025**
(21) Application number: 23897821.7
(22) Date of filing: 29.11.2023
(51) Int. Cl.: A23L 2/56, A23L 2/00, A23L 27/00, A61K 47/10, A61K 47/12

(54) **ORAL COMPOSITION**

(30) Priority: 30.11.2022 JP 2022192321
(71) Applicant: Kao Corporation, Chuo-ku, Tokyo 103-8210 (JP)
(72) Inventor: YANO, Michiko, Tokyo 131-8501 (JP); KANARI, Haruna, Tokyo 131-8501 (JP); NIWA, Sachiko, Tokyo 131-8501 (JP)
(74) Representative: Hoffmann Eitle
(86) International application number: PCT/JP2023/042723
(87) International publication number: WO 2024/117174

(57) **Abstract**

Provided is an oral composition suppressed in unpleasant odor of a branched fatty acid having 6 or less carbon atoms. The oral composition includes the following components (A) and (B): (A) 0.01 mass ppm or more of a branched fatty acid having 6 or less carbon atoms; and (B) 0.05 mass ppm or more of at least one monoterpene alcohol selected from the group consisting of linalool and geraniol.

## Description

### Field of the Invention

The present invention relates to an oral composition, an unpleasant odor suppressant for a branched fatty acid having 6 or less carbon atoms, and a method of suppressing an unpleasant odor thereof.

### Background of the Invention

A short-chain fatty acid having a branched chain having 6 or less carbon atoms naturally occurs as a component of an essential oil in a free or ester form. Such fatty acid is used as an aroma component for a perfume at an extremely low concentration. In addition, the short-chain fatty acid having a branched chain has been known to be produced by intestinal bacteria in a human large intestine in the same manner as in a linear short-chain fatty acid. The short-chain fatty acid is considered to have an action similar to that of the linear short-chain fatty acid, though much still remains to be known about its physiological action. Meanwhile, the short-chain fatty acid having a branched chain has been known to as a causative substance for an unpleasant odor generated from the respective parts of the whole body and an off-flavor odor present in a food, and has a unique odor, and hence its suppression is desired.

Linalool and geraniol are one of monoterpene alcohols, respectively, and have each been widely used for blending various flower oils, or as a cosmetic perfume or a food perfume. For example, there are conventional reports that a beer-taste beverage that has a citrus-like aroma and is suppressed in deterioration of a flavor and occurrence of an unpleasant odor due to storage is produced by adding linalool, geraniol, and the like to the beer-taste beverage (Patent Document 1), and that a beverage having an unprecedented pleasant aroma is produced by adding linalool and geraniol to a sparkling beverage (Patent Document 2).

Further, there is a report that, in the production of a beverage containing linalool and geraniol, a flavor of the beverage is enhanced by adding a lower fatty acid or an ester thereof (Patent Document 3). However, it has not been known what kind of action each of linalool and geraniol has on the unpleasant odor of the short-chain fatty acid having a branched chain.

[Patent Document 1] JP-A-2020-96569
[Patent Document 2] JP-A-2017-143808
[Patent Document 3] JP-A-2016-25866

### Summary of the Invention

The present invention relates to the following (1) to (3).
(1) An oral composition, comprising the following components (A) and (B):
   (A) 0.01 mass ppm or more of a branched fatty acid having 6 or less carbon atoms; and
   (B) 0.05 mass ppm or more of at least one monoterpene alcohol selected from the group consisting of linalool and geraniol.
(2) An unpleasant odor suppressant for a branched fatty acid having 6 or less carbon atoms, the unpleasant odor suppressant comprising at least one monoterpene alcohol selected from the group consisting of linalool and geraniol as an active ingredient.
(3) A method of suppressing an unpleasant odor of a branched fatty acid having 6 or less carbon atoms, the method comprising causing at least one monoterpene alcohol selected from the group consisting of linalool and geraniol to coexist with respect to a branched fatty acid having 6 or less carbon atoms.

### Detailed Description of the Invention

The present invention relates to providing an oral composition suppressed in unpleasant odor of a branched fatty acid having 6 or less carbon atoms, an unpleasant odor suppressant, and a method of suppressing an unpleasant odor.

The present inventors made extensive investigations. As a result, the inventors found that linalool and/or geraniol had a suppressing action on an unpleasant odor of a branched fatty acid having 6 or less carbon atoms, and that the incorporation of linalool and/or geraniol provided an oral composition suppressed in unpleasant odor of a branched fatty acid having 6 or less carbon atoms.

The present invention can provide the oral composition suppressed in unpleasant odor of a branched fatty acid having 6 or less carbon atoms, the unpleasant odor suppressant, and the method of suppressing an unpleasant odor.

### [Oral Composition]

The oral composition of the present invention comprises a branched fatty acid having 6 or less carbon atoms as a component (A). The number of carbon atoms of the branched fatty acid is 6 or less, preferably from 2 to 6, more preferably 4 or 5, even more preferably 5. The number of branches and positions of the branches of the branched fatty acid are not particularly limited, but an iso-fatty acid having a methyl branch on the second carbon atom from a terminal methyl group and an anteiso-fatty acid having a methyl branch on the third carbon atom from the terminal methyl group are preferred. The branched fatty acid may be any one of a saturated fatty acid or an unsaturated fatty acid.

Specific examples of the component (A) include isobutyric acid, isovaleric acid, 2-methylbutyric acid, 4-methylpentanoic acid, and 4-methyl-3-pentenoic acid. Of those, one or more selected from the group consisting of isobutyric acid, 2-methylbutyric acid, and isovaleric acid are preferred from the viewpoint that the effect of the present invention is easily obtained, and isovaleric acid is more preferred.

The components (A) may be used alone or in combination thereof.

As the component (A), a commercial reagent may be used, or an extract of a plant rich in the component (A) may be used. When the plant extract is used as the component (A), an extraction method and extraction conditions for the plant extract are not particularly limited, and a known method may be adopted.

While the plant is not particularly limited as long as the plant contains the component (A), coffee beans are preferred. The coffee beans are preferably one or more selected from the group consisting of green coffee beans and lightly roasted coffee beans, more preferably green coffee beans. As used herein, the term "lightly roasted coffee beans" refers to roasted coffee beans each having an L value of 30 or more and 60 or less, and from the viewpoints of the suppression of the unpleasant odor of the component (A) and the physiological effect thereof, the L value is preferably 32 or more, more preferably 34 or more, more preferably 36 or more, more preferably 38 or more, even more preferably 40 or more. The kind and production site of the coffee beans are not particularly limited. In addition, the term "L value" as used herein refers to a value obtained by measuring the brightness of roasted coffee beans with a color-difference meter while setting a black color and a white color to an L value of 0 and an L value of 100, respectively.

While the content of the component (A) in the oral composition of the present invention is 0.01 mass ppm or more, the content is preferably 0.05 mass ppm or more, more preferably 0.3 mass ppm or more from the viewpoint that the effect of the present invention is more easily obtained, and is preferably 30 mass ppm or less, more preferably 20 mass ppm or less, more preferably 12 mass ppm or less, even more preferably 8 mass ppm or less from the viewpoint of suppressing the unpleasant odor of the component (A). Moreover, the content of the component (A) in the oral composition of the present invention is 0.01 mass ppm or more, preferably from 0.01 mass ppm to 30 mass ppm, more preferably from 0.01 mass ppm to 20 mass ppm, more preferably from 0.05 mass ppm to 12 mass ppm, even more preferably from 0.3 mass ppm to 8 mass ppm.

The content of the component (A) may be measured by an analysis method suitable for the state of a measurement sample among generally known measurement methods, and for example, may be measured by a GC/MS method or a HPLC method. In addition, its analysis may be requested to a third-party organization. The analysis by the GC/MS method may be submitted to, for example, Japan Food Research Laboratories, and the analysis by the HPLC method may be submitted to, for example, Shimadzu Techno-Research, Inc.

The oral composition of the present invention comprises at least one monoterpene alcohol selected from the group consisting of linalool and geraniol as a component (B). Linalool and geraniol are each a compound represented by the molecular formula C₁₀H₁₈O, and are in an isomeric relationship.

Linalool has optical isomers. Linalool may be any one of the optical isomers, or a mixture of the optical isomers.

As the component (B), a commercial reagent may be used, or an extract of a plant rich in the component (B) may be incorporated. In addition, the component (B) may be synthesized by organic synthesis. The plant may be appropriately selected without departing from the gist of the present invention as long as the plant contains the component (B) and is typically used in the field of a food and beverage. In addition, an extraction method and extraction conditions are not particularly limited, and a known method may be adopted.

Linalool and geraniol may be used alone or in combination thereof.

While the content of the component (B) in the oral composition of the present invention is 0.05 mass ppm or more from the viewpoint of suppressing the unpleasant odor of the component (A), the content is preferably 0.08 mass ppm or more, more preferably 8 mass ppm or more, even more preferably 80 mass ppm or more. In addition, from the viewpoint of further improving the flavor, and taste and flavor, of the oral composition, the content is preferably 3 000 mass ppm or less, more preferably 2 000 mass ppm or less, even more preferably 1 200 mass ppm or less. Moreover, the content of the component (B) in the oral composition of the present invention is 0.05 mass ppm or more, preferably from 0.05 mass ppm to 3 000 mass ppm, more preferably from 0.08 mass ppm to 2 000 mass ppm, more preferably from 8 mass ppm to 2 000 mass ppm, even more preferably from 80 mass ppm to 1 200 mass ppm.

When linalool is used as the component (B), from the viewpoint of suppressing the unpleasant odor of the component (A), the content of the component (B) in the oral composition is 0.05 mass ppm or more, preferably 0.08 mass ppm or more, more preferably 0.8 mass ppm or more, more preferably 8 mass ppm or more, even more preferably 80 mass ppm or more. In addition, from the viewpoint of further improving the flavor, and taste and flavor, of the oral composition, the content is preferably 3 000 mass ppm or less, more preferably 2 000 mass ppm or less, even more preferably 1 200 mass ppm or less. Moreover, the content of linalool serving as the component (B) in the oral composition of the present invention is 0.05 mass ppm or more, preferably from 0.05 mass ppm to 3 000 mass ppm, more preferably from 0.08 mass ppm to 2 000 mass ppm, more preferably from 0.8 mass ppm to 2 000 mass ppm, more preferably from 8 mass ppm to 2 000 mass ppm, even more preferably from 80 mass ppm to 1 200 mass ppm.

In addition, when geraniol is used as the component (B), from the viewpoint of suppressing the unpleasant odor of the component (A), the content of the component (B) in the oral composition is 0.05 mass ppm or more, preferably 0.08 mass ppm or more, more preferably 8 mass ppm or more, even more preferably 800 mass ppm or more. In addition, from the viewpoint of further improving the flavor, and taste and flavor, of the oral composition, the content is preferably 3 000 mass ppm or less, more preferably 2 000 mass ppm or less, even more preferably 1 500 mass ppm or less. Moreover, the content of geraniol serving as the component (B) in the oral composition of the present invention is 0.05 mass ppm or more, preferably from 0.05 mass ppm to 3 000 mass ppm, more preferably from 0.08 mass ppm to 2 000 mass ppm, more preferably from 8 mass ppm to 2 000 mass ppm, even more preferably from 800 mass ppm to 1 500 mass ppm.

The content of the component (B) may be measured by an analysis method suitable for the state of a measurement sample among generally known measurement methods, and may be measured by, for example, a GC/MS method. A specific example thereof may be a method described in Examples to be described later. At the time of the measurement, such treatment as described below may be appropriately performed as required: the sample is lyophilized so that the sample may be adapted to the detection range of an apparatus; or contaminants in the sample are removed so that the sample may be adapted to the resolution of the apparatus.

In the oral composition of the present invention, from the viewpoint of suppressing the unpleasant odor of the component (A), a mass ratio of the component (B) to the component (A), [(B)/(A)], is preferably 0.003 or more, more preferably 0.15 or more, more preferably 15 or more, even more preferably 100 or more. In addition, from the viewpoint of further improving the flavor, and taste and flavor, of the oral composition, the mass ratio is preferably 20 000 or less, more preferably 16 000 or less, even more preferably 12 000 or less. Moreover, in the present invention, the mass ratio of the component (B) to the component (A), [(B)/(A)], is preferably from 0.003 to 20 000, more preferably from 0.15 to 20 000, more preferably from 15 to 16 000, even more preferably from 100 to 12 000.

When linalool is used as the component (B), from the viewpoint of suppressing the unpleasant odor of the component (A), a mass ratio of linalool to the component (A), [linalool/(A)], in the oral composition of the present invention is preferably 0.003 or more, more preferably 0.15 or more, more preferably 1 or more, more preferably 15 or more, more preferably 80 or more, even more preferably 150 or more. In addition, from the viewpoint of further improving the flavor, and taste and flavor, of the oral composition, the mass ratio is preferably 20 000 or less, more preferably 16 000 or less, more preferably 12 000 or less, more preferably 6 000 or less, even more preferably 3 000 or less. Moreover, in the present invention, the mass ratio of linalool serving as the component (B) to the component (A), [linalool/(A)], is preferably from 0.003 to 20 000, more preferably from 0.15 to 20 000, more preferably from 1 to 20 000, more preferably from 15 to 16 000, more preferably from 80 to 12 000, more preferably from 80 to 6 000, even more preferably from 150 to 3 000.

In addition, when geraniol is used as the component (B), from the viewpoint of suppressing the unpleasant odor of the component (A), a mass ratio of geraniol to the component (A), [geraniol/(A)], in the oral composition of the present invention is preferably 0.003 or more, more preferably 0.15 or more, more preferably 1 or more, more preferably 15 or more, even more preferably 100 or more. In addition, from the viewpoint of further improving the flavor, and taste and flavor, of the oral composition, the mass ratio is preferably 20 000 or less, more preferably 16 000 or less, more preferably 12 000 or less, more preferably 6 000 or less, even more preferably 3 000 or less. Moreover, in the present invention, the mass ratio of geraniol serving as the component (B) to the component (A), [geraniol/(A)], is preferably from 0.003 to 20 000, more preferably from 0.15 to 20 000, more preferably from 1 to 16 000, more preferably from 15 to 12 000, more preferably from 15 to 6 000, even more preferably from 100 to 3 000.

The oral composition of the present invention may comprise one or more kinds of additives, such as a sweetener, an acidulant, an amino acid, a protein, a vitamin, a mineral, a plant extract, an ester, a coloring matter, an emulsifier, a milk component, a preservative, a seasoning, a quality stabilizer, and a perfume, as desired. The content of the additive may be appropriately set within a range that does not impair the purpose of the present invention.

The term "oral composition" as used herein refers to a composition that is unlikely to cause harm to human health and is exclusively orally ingested in a general social life, and the composition is not limited by divisions, such as a food, a pharmaceutical, and a quasi-drug, in administrative divisions. Accordingly, the oral composition of the present invention means a composition to be orally ingested, such as encompassing a wide variety of foods and beverages for forming, for example, a general food, a health food (functional food and beverage), a food with health claims (a food for specified health use, a food with nutrient function claims, or a food with function claims), a quasi-drug, and a pharmaceutical.

The oral composition of the present invention may be a solid or a liquid at normal temperature (20°C±15°C), and may adopt any appropriate form. A suitable aspect of the oral composition of the present invention may be, for example, a solid oral composition or a liquid oral composition.

The oral composition of the present invention is preferably used for an oral composition excluding a coffee beverage. The coffee beverage is a beverage for savoring a roasted aroma specific to roasted coffee beans, and the unpleasant odor of the component (A) may be masked with a roasted odor of the roasted coffee beans. Accordingly, the problem of the unpleasant odor of the component (A) of the present invention may hardly occur.

The coffee beverage may be clearly distinguished as a product from the oral composition of the present invention because the content of (C) furfuryl mercaptan (hereinafter referred to as "component (C)") in the coffee beverage is typically 0.00006 mass% or more. That is, the content of the component (C) in the oral composition of the present invention is preferably less than 0.00006 mass%, more preferably less than 0.00003 mass%, more preferably less than 0.00001 mass%, and the composition is even more preferably substantially free of the component (C). As used herein, the phrase "substantially free of" is a concept encompassing not only a case in which the component (C) is completely absent in the oral composition but also a case in which its concentration is less than a detection limit. The content of the component (C) may be measured by an analysis method suitable for the state of a measurement sample among generally known measurement methods, and may be measured by, for example, a GC/MS method. At the time of the measurement, such treatment as described below may be appropriately performed as required: the sample is lyophilized so that the sample may be adapted to the detection range of an apparatus; or contaminants in the sample are removed so that the sample may be adapted to the resolution of the apparatus.

In addition, the coffee beverage is typically a beverage prepared by using roasted coffee beans including roasted coffee beans each having an L value of less than 30. Accordingly, the present invention is directed to an oral composition excluding a coffee beverage prepared by using roasted coffee beans including roasted coffee beans each having an L value of preferably less than 30, more preferably less than 32, more preferably less than 34, more preferably less than 36, more preferably less than 38, even more preferably less than 40. The roasted coffee beans may be roasted coffee beans from which 90% or more of caffeine has been removed. In addition, the content of a coffee content in the coffee beverage includes a coffee content extracted or eluted from 1 g or more of roasted coffee beans in terms of green coffee beans in 100 g of a net content. The term "value in terms of green beans" as used herein means that 1 g of roasted coffee beans correspond to 1.3 g of green coffee beans (Newly Revised Edition·Soft Drinks, supervision: Japan Soft Drink Association, publication: Korin, published on December 25, 1989, described on page 421). The kind of the coffee beverage is not particularly limited, but examples thereof may include coffee beverages and the like defined in Article 2 of "Fair Competition Code on Labeling of Coffee Beverages and the Like" amended and enforced on August 19, 2019, that is, "coffee", a "coffee beverage," a "coffee-containing soft drink," and a "coffee-containing carbonated beverage."

The solid oral composition of the present invention may be a solid that can be orally ingested as it is. Examples of the form thereof may include a powder form, a granule form, a tablet form, a rod form, a plate form, and a block form. A solid content in the solid oral composition of the present invention is typically 90 mass% or more, preferably 93 mass% or more, more preferably 95 mass% or more, even more preferably 97 mass% or more. The upper limit of such solid content is not particularly limited, and the solid content may be 100 mass%. As used herein, the term "solid content" refers to the mass of a residue obtained by drying a sample in an electric thermostat dryer at 105°C for 3 hours to remove its volatile substance.

Examples of the solid oral composition of the present invention may include a food, a pharmaceutical, and a quasi-drug. Of those, a solid food is preferred because the effect of the present invention is easily obtained, and a powder food is more preferred.

When the solid oral composition of the present invention is a solid food, examples thereof may include: confectionery, such as syrup solid, candy, gum, chocolate, and cookie bread; and health, beauty, and dietary supplements such as a supplement.

In addition, when the solid oral composition of the present invention is a pharmaceutical or a quasi-drug, examples of the dosage form thereof may include a granule, a powder, a tablet, a pill, a chewable, and a troche. In addition, when the composition is used as a tablet, a dividable tablet having formed therein a dividing line is permitted.

Of those, a supplement, a powder, a tablet, or a granule is preferred as the solid oral composition.

The solid oral composition of the present invention may comprise an acceptable carrier as required so as to be formed into a solid form. Examples thereof may include carriers including: excipients (e.g., starches, such as corn starch (corn), potato starch (potato), sweet potato starch (sweet potato), and tapioca starch; dextrin; sugar alcohols, such as xylitol, sorbitol, maltitol, lactitol, reduced palatinose, trehalose, and palatinose; lactose; oligosaccharides; crystalline cellulose; light silicic anhydride; and calcium hydrogen phosphate); binders (e.g., hydroxypropylmethylcellulose, hydroxypropylcellulose, gelatin, pregelatinized starch, polyvinylpyrrolidone, polyvinyl alcohol, pullulan, methylcellulose, and hydrogenated oils); disintegrants (e.g., carmellose, carmellose calcium, croscarmellose sodium, and crospovidone); lubricants (e.g., calcium stearate, magnesium stearate, sucrose fatty acid esters, sodium stearyl fumarate, talc, and silicon dioxide); fluidity improvers; taste-making agents (e.g., stevia); extenders; surfactants; dispersants; buffers; antioxidants; preservatives; quality stabilizers; and diluents. Of those, a carrier free of any unpleasant odor is preferably selected and used. For example, dextrin, maltitol, or lactose is suitably used as the excipient because such excipient is free of any unpleasant odor.

In addition, the solid oral composition of the present invention may be an instant beverage composition. As used herein, the term "instant beverage composition" refers to a composition that is diluted with a liquid in accordance with a predetermined usage method and orally ingested as a reconstituted beverage. The liquid is not particularly limited as long as the liquid can reconstitute the composition into a beverage. Examples of the liquid may include water, carbonated water, cow's milk, and soy milk. The temperature of the liquid is not limited. A dilution factor, which has only to follow a predetermined usage method, is typically from 30 mass-fold to 800 mass-fold, preferably from 80 mass-fold to 600 mass-fold.

When the oral composition of the present invention is in a form of a solid or a concentrate, such as an instant beverage composition, the contents of the component (A) and the component (B) have only to satisfy the above-mentioned requirements, and preferably, the mass ratio [(B)/(A)] has only to satisfy the above-mentioned requirements, at the time of the dilution of the composition by a predetermined dilution factor.

The solid oral composition of the present invention may be produced in accordance with a conventional method, and an appropriate method may be adopted. The composition may be produced by, for example, mixing the component (A) and the component (B), and as required, any other component so that the contents of the component (A) and the component (B) may fall within the above-mentioned ranges. The order in which the component (A) and the component (B) are mixed is not particularly limited, and one of the components may be added to the other, or both the components may be simultaneously added. While an appropriate method, such as stirring or shaking, may be adopted as a method for the mixing, a mixing apparatus may be used. The mixing system of the mixing apparatus may be of a rotating vessel type or a fixed vessel type. As the rotating vessel type, for example, a horizontal cylinder type, a V type, a double-cone type, or a cubic type may be adopted. In addition, as the fixed vessel type, for example, a ribbon type, a screw type, a conical screw type, a paddle type, a fluidized bed type, or a Phillips blender may be adopted.

In addition, the solid oral composition of the present invention may be produced as a granulated product by a known granulation method. Examples of the granulation method may include spray granulation, fluidized bed granulation, compression granulation, tumbling granulation, stirring granulation, extrusion granulation, and powder coating granulation. Granulation conditions may be appropriately selected in accordance with the granulation method. In addition, when the solid oral composition is produced as a tablet, any of wet tableting and dry tableting may be adopted, and a known compression molding machine may be used.

The solid oral composition of the present invention may be loaded into a package. Examples of the package may include a bottle, a can, a box-type container, a stick-type package, and a pillow-type package. When the solid oral composition of the present invention is loaded into the package, a commercial loading machine may be used. For example, small portions each corresponding to one intake of the solid oral composition of the present invention may be individually packaged. When the solid oral composition is an instant beverage composition, the composition may be, for example, any one of the following: a product that is filled in a container such as a bottle and is measured with a spoon or the like in an amount corresponding to a cup before drinking; a cup type accommodating an amount corresponding to a cup; and a stick type in which small portions each corresponding to a cup are individually packaged.

The inside of the container and the inside of a packaging material may be filled with a nitrogen gas, and the packaging material preferably has low oxygen permeability from the viewpoint of quality maintenance.

The form of the liquid oral composition of the present invention is not particularly limited as long as the composition has fluidity at normal temperature (20°C±15°C). Examples thereof may include a liquid, a concentrated liquid form, a gel form, and a jelly form.

Examples of the product form of the liquid oral composition of the present invention may include: a ready-to-drink (RTD)-type beverage composition; an instant beverage composition; dairy products, such as yogurt, processed milk, and fermented milk; an oil and fat, and an oil-and-fat processed food, such as a salad oil, a tempura oil, margarine, mayonnaise, shortening, whipped cream, and dressing; seasonings, such as sauce and tare sauce; and health, beauty, and dietary supplements such as a drinkable preparation. As used herein, the term "RTD-type beverage composition" refers to a beverage that can be drunk as it is without being diluted.

Of those, an RTD-type beverage composition is preferred as the liquid oral composition. Examples of the form of the RTD-type beverage composition may include a liquid, a concentrated liquid form, a gel form, and a jelly form. When the form is a concentrated liquid form, a gel form, or a jelly form, the beverage composition has only to be capable of being sucked through a suction opening provided in its container or a straw, and its solid content concentration is not particularly limited.

From the viewpoint of its taste and flavor, the pH (20°C) of the RTD-type beverage composition is preferably 3 or more, more preferably 3.5 or more, even more preferably 4 or more, and is preferably 7 or less, more preferably 6.5 or less, even more preferably 6 or less. The pH is measured with a pH meter after the temperature of the composition has been adjusted to 20°C.

The RTD-type beverage composition may be a non-alcoholic beverage or an alcoholic beverage. As used herein, the term "non-alcoholic beverage" refers to a beverage having an alcohol concentration of less than 1 v/v%, and encompasses a beverage that is completely free of any alcohol and a beverage having an alcohol concentration of 0.00 v/v%. The term "alcohol" as used herein means ethanol unless otherwise stated.

Examples of the non-alcoholic beverage may include a tea beverage, a carbonated beverage, a fruit juice beverage, a vegetable beverage, a dairy beverage, a sports beverage, an isotonic beverage, enhanced water, bottled water, near water, a nutritional drinkable preparation, and a beauty drinkable preparation.

Examples of the alcoholic beverage may include beer, wine, Japanese sake, plum liquor, sparkling liquor, whiskey, brandy, Japanese distilled spirit, rum, gin, and liqueurs.

The RTD-type beverage composition may be packed in a container. The container is not particularly limited as long as the container is a typical packaging container, and examples thereof may include: a molded container containing polyethylene terephthalate as a main component (so-called plastic bottle); a metal can; a paper container composited with metal foil or a plastic film; and a bottle.

When the RTD-type beverage composition is a container-packed beverage composition, the composition may have already been sterilized by heating. A method for the heat sterilization is not particularly limited as long as the method conforms to conditions specified in laws and regulations to be applied (the Food Sanitation Act in Japan).

The liquid oral composition of the present invention may be produced in accordance with a conventional method, and any appropriate method may be adopted. The composition may be produced by, for example, mixing the component (A) and the component (B), and as required, any other component together with a liquid so that the contents of the component (A) and the component (B) may fall within the above-mentioned ranges. The order in which the component (A) and the component (B), and the other component are mixed is not particularly limited, and the components may be added in any order. Examples of the liquid may include water, carbonated water, cow's milk, and soy milk. The temperature of the liquid is not limited.

### [Unpleasant Odor Suppressant for Branched Fatty Acid having 6 or less Carbon Atoms and Method of suppressing Unpleasant Odor thereof]

Each of the unpleasant odor suppressant and method of suppressing an unpleasant odor of the present invention uses (B) at least one monoterpene alcohol selected from the group consisting of linalool and geraniol as an active ingredient, and is exclusively used in the suppression of the unpleasant odor of (A) the branched fatty acid having 6 or less carbon atoms. The specific configurations of (A) the branched fatty acid having 6 or less carbon atoms and (B) the at least one monoterpene alcohol selected from the group consisting of linalool and geraniol are as described above.

In each of the unpleasant odor suppressant and method of suppressing an unpleasant odor of the present invention, (A) the branched fatty acid having 6 or less carbon atoms, and (B) the at least one monoterpene alcohol selected from the group consisting of linalool and geraniol have only to be caused to coexist. In that case, 0.05 mass ppm or more of (B) the at least one monoterpene alcohol selected from the group consisting of linalool and geraniol is preferably caused to coexist with respect to 0.01 mass ppm or more of (A) the branched fatty acid having 6 or less carbon atoms from the viewpoint of suppressing the unpleasant odor.

In addition, the unpleasant odor suppressant of the present invention may be applied not only to the component (A) but also to an oral product comprising the component (A).

The oral product is not particularly limited as long as the product can be orally ingested, and the product may be a liquid or a solid. The oral product may be, for example, a food and beverage, a pharmaceutical, or a quasi-drug comprising the component (A). Of those, a food and beverage is preferred.

The food and beverage may be, for example, a solid food comprising the component (A), or a beverage or an instant beverage comprising the component (A). The food and beverage may be produced in accordance with a conventional method depending on the kind of the food and beverage.

The dosage form of each of the pharmaceutical and the quasi-drug is not particularly limited, and may, for example, a preparation for oral administration. For example, a known dosage form, such as a solution or syrup, may be adopted. In addition, when the pharmaceutical or the quasi-drug is turned into the preparation, a known additive may be blended. The pharmaceutical and the quasi-drug may each be produced in accordance with a conventional method.

The respective contents of the component (A) and the component (B) in the oral product, and the mass ratio [(B)/(A)] are as described above.

In relation to the above-mentioned embodiments, the present invention further discloses the following oral composition, unpleasant odor suppressant for a branched fatty acid having 6 or less carbon atoms, and method of suppressing an unpleasant odor thereof.

<1> An oral composition, comprising the following components (A) and (B):
   (A) 0.01 mass ppm or more of a branched fatty acid having 6 or less carbon atoms; and
   (B) 0.05 mass ppm or more of at least one monoterpene alcohol selected from the group consisting of linalool and geraniol.
<2> The oral composition according to <1>, wherein the component (A) is preferably one or more selected from the group consisting of isobutyric acid, 2-methylbutyric acid, and isovaleric acid, more preferably isovaleric acid.
<3> The oral composition according to <1> or <2>, wherein the component (A) is preferably derived from an extract of one or more coffee beans selected from the group consisting of green coffee beans and lightly roasted coffee beans.
<4> The oral composition according to any one of <1> to <3>, wherein a content of the component (A) is 0.01 mass ppm or more, preferably 0.05 mass ppm or more, more preferably 0.3 mass ppm or more, and is preferably 30 mass ppm or less, more preferably 20 mass ppm or less, more preferably 12 mass ppm or less, even more preferably 8 mass ppm or less.
<5> The oral composition according to any one of <1> to <3>, wherein a content of the component (A) is 0.01 mass ppm or more, preferably from 0.01 mass ppm to 30 mass ppm, more preferably from 0.01 mass ppm to 20 mass ppm, more preferably from 0.05 mass ppm to 12 mass ppm, even more preferably from 0.3 mass ppm to 8 mass ppm.
<6> The oral composition according to any one of <1> to <5>, wherein a content of the component (B) is 0.05 mass ppm or more, preferably 0.08 mass ppm or more, more preferably 8 mass ppm or more, even more preferably 80 mass ppm or more, and is preferably 3 000 mass ppm or less, more preferably 2 000 mass ppm or less, even more preferably 1 200 mass ppm or less.
<7> The oral composition according to any one of <1> to <5>, wherein a content of the component (B) is 0.05 mass ppm or more, preferably from 0.05 mass ppm to 3 000 mass ppm, more preferably from 0.08 mass ppm to 2 000 mass ppm, more preferably from 8 mass ppm to 2 000 mass ppm, even more preferably from 80 mass ppm to 1 200 mass ppm.
<8> The oral composition according to any one of <1> to <5>, wherein the component (B) is linalool, and a content thereof is 0.05 mass ppm or more, preferably 0.08 mass ppm or more, more preferably 0.8 mass ppm or more, more preferably 8 mass ppm or more, even more preferably 80 mass ppm or more, and is preferably 3 000 mass ppm or less, more preferably 2 000 mass ppm or less, even more preferably 1 200 mass ppm or less.
<9> The oral composition according to any one of <1> to <5>, wherein the component (B) is linalool, and a content thereof is 0.05 mass ppm or more, preferably from 0.05 mass ppm to 3 000 mass ppm, more preferably from 0.08 mass ppm to 2 000 mass ppm, more preferably from 0.8 mass ppm to 2 000 mass ppm, more preferably from 8 mass ppm to 2 000 mass ppm, even more preferably from 80 mass ppm to 1 200 mass ppm.
<10> The oral composition according to any one of <1> to <5>, wherein the component (B) is geraniol, and a content thereof is 0.05 mass ppm or more, preferably 0.08 mass ppm or more, more preferably 8 mass ppm or more, even more preferably 800 mass ppm or more, and is preferably 3 000 mass ppm or less, more preferably 2 000 mass ppm or less, even more preferably 1 500 mass ppm or less.
<11> The oral composition according to any one of <1> to <5>, wherein the component (B) is geraniol, and a content thereof is 0.05 mass ppm or more, preferably from 0.05 mass ppm to 3 000 mass ppm, more preferably from 0.08 mass ppm to 2 000 mass ppm, more preferably from 8 mass ppm to 2 000 mass ppm, even more preferably from 800 mass ppm to 1 500 mass ppm.
<12> The oral composition according to any one of <1> to <11>, wherein a mass ratio of the component (B) to the component (A), [(B)/(A)], is preferably 0.003 or more, more preferably 0.15 or more, more preferably 15 or more, even more preferably 100 or more, and is preferably 20 000 or less, more preferably 16 000 or less, even more preferably 12 000 or less.
<13> The oral composition according to any one of <1> to <11>, wherein a mass ratio of the component (B) to the component (A), [(B)/(A)], is preferably from 0.003 to 20 000, more preferably from 0.15 to 20 000, more preferably from 15 to 16 000, even more preferably from 100 to 12 000.
<14> The oral composition according to any one of <1> to <9>, wherein when linalool is used as the component (B), a mass ratio of linalool to the component (A), [linalool/(A)], is preferably 0.003 or more, more preferably 0.15 or more, more preferably 1 or more, more preferably 15 or more, more preferably 80 or more, even more preferably 150 or more, and is preferably 20 000 or less, more preferably 16 000 or less, more preferably 12 000 or less, more preferably 6 000 or less, even more preferably 3 000 or less.
<15> The oral composition according to any one of <1> to <9>, wherein when linalool is used as the component (B), a mass ratio of linalool to the component (A), [linalool/(A)], is preferably from 0.003 to 20 000, more preferably from 0.15 to 20 000, more preferably from 1 to 20 000, more preferably from 15 to 16 000, more preferably from 80 to 12 000, more preferably from 80 to 6 000, even more preferably from 150 to 3 000.
<16> The oral composition according to any one of <1> to <7>, <10>, and <11>, wherein when geraniol is used as the component (B), a mass ratio of geraniol to the component (A), [geraniol/(A)], is preferably 0.003 or more, more preferably 0.15 or more, more preferably 1 or more, more preferably 15 or more, even more preferably 100 or more, and is preferably 20 000 or less, more preferably 16 000 or less, more preferably 12 000 or less, more preferably 6 000 or less, even more preferably 3 000 or less.
<17> The oral composition according to any one of <1> to <7>, <10>, and <11>, wherein when geraniol is used as the component (B), a mass ratio of geraniol to the component (A), [geraniol/(A)], is preferably from 0.003 to 20 000, more preferably from 0.15 to 20 000, more preferably from 1 to 16 000, more preferably from 15 to 12 000, more preferably from 15 to 6 000, even more preferably from 100 to 3 000.
<18> The oral composition according to any one of <1> to <17>, wherein the oral composition is preferably a solid oral composition or a liquid oral composition.
<19> The oral composition according to any one of <1> to <18>, wherein the oral composition is preferably an instant beverage composition.
<20> The oral composition according to any one of <1> to <19>, wherein the oral composition is preferably an oral composition excluding a coffee beverage.
<21> The oral composition according to any one of <1> to <20>, wherein a content of furfuryl mercaptan is preferably less than 0.00006 mass%, more preferably less than 0.00003 mass%, more preferably less than 0.00001 mass%, and the oral composition is even more preferably substantially free of the furfuryl mercaptan.
<22> The oral composition according to any one of <1> to <21>, wherein the oral composition is an oral composition excluding a coffee beverage prepared by using roasted coffee beans including roasted coffee beans each having an L value of preferably less than 30, more preferably less than 32, more preferably less than 34, more preferably less than 36, more preferably less than 38, even more preferably less than 40.
<23> An unpleasant odor suppressant for a branched fatty acid having 6 or less carbon atoms, the unpleasant odor suppressant comprising at least one monoterpene alcohol selected from the group consisting of linalool and geraniol as an active ingredient.
<24> A method of suppressing an unpleasant odor of a branched fatty acid having 6 or less carbon atoms, the method comprising causing at least one monoterpene alcohol selected from the group consisting of linalool and geraniol to coexist with respect to the branched fatty acid having 6 or less carbon atoms.
<25> The unpleasant odor suppressant according to <23> or the method of suppressing an unpleasant odor according to <24>, wherein the branched fatty acid having 6 or less carbon atoms is preferably one or more selected from the group consisting of isobutyric acid, 2-methylbutyric acid, and isovaleric acid, more preferably isovaleric acid.
<26> The unpleasant odor suppressant or the method of suppressing an unpleasant odor according to any one of <23> to <25>, wherein the branched fatty acid having 6 or less carbon atoms is preferably derived from an extract of one or more coffee beans selected from the group consisting of green coffee beans and lightly roasted coffee beans.
<27> The unpleasant odor suppressant or the method of suppressing an unpleasant odor according to any one of <23> to <26>, wherein a mass ratio [monoterpene alcohol/branched fatty acid having 6 or less carbon atoms] of the monoterpene alcohol to the branched fatty acid having 6 or less carbon atoms is preferably 0.15 or more, more preferably 15 or more, even more preferably 100 or more, and is preferably 20 000 or less, more preferably 16 000 or less, even more preferably 12 000 or less.

### Examples

### (1) Analysis of Branched Fatty Acid

A branched fatty acid may be analyzed in a third-party organization by a GC/MS method or a HPLC method. The analysis by the GC/MS method may be submitted to Japan Food Research Laboratories, and the analysis by the HPLC method may be submitted to Shimadzu Techno-Research, Inc.

### (2) Analysis of Linalool and Geraniol

Linalool and geraniol may each be analyzed in a third-party organization by a GC/MS method, and the analysis may be submitted to Japan Food Research Laboratories.

### Examples 1 to 5 and Comparative Examples 1 and 2

Isovaleric acid and linalool were dissolved in 99.5% ethanol (manufactured by FUJIFILM Wako Pure Chemical Corporation) at the respective concentrations, and 1 g of maltitol powder (Lesys fine powder; manufactured by Mitsubishi Corporation Life Sciences Limited) and 100 uL of each of the ethanol solutions were mixed. The concentrations of the ethanol solutions were adjusted so that the final concentrations shown in Table 2 were achieved. The resultant was left to stand still at 50°C for 10 minutes so that ethanol was volatilized. Thus, powdered oral compositions were prepared. The respective oral compositions were subjected to analysis and sensory evaluation. The results are collectively shown in Table 2.

### [Sensory Evaluation]

A sensory test for the "unpleasant odor intensity of isovaleric acid" of the powdered oral composition obtained in each of Examples and Comparative Examples was performed by two expert panelists in accordance with the following procedure. First, "isovaleric acid standard powders" whose concentrations had been adjusted in advance so that the intensity of the "unpleasant odor of isovaleric acid" changed in 5 stages at equal intervals were prepared by using an isovaleric acid reagent. The "isovaleric acid standard powders" were prepared as follows: isovaleric acid was dissolved in 99.5% ethanol (manufactured by FUJIFILM Wako Pure Chemical Corporation) at the respective concentrations; and 1 g of maltitol powder (Lesys fine powder; manufactured by Mitsubishi Corporation Life Sciences Limited) and 100 uL of each of the ethanol solutions were mixed. The concentrations of the ethanol solutions were adjusted so that the final concentrations shown in Table 1 were achieved. The resultant was left to stand still at 50°C for 10 minutes so that ethanol was volatilized. After that, an odor was evaluated by two expert panelists. The evaluation was performed as described below. The respective expert panelists agreed that the unpleasant odor intensities of isovaleric acid at the respective concentrations were given scores shown in Table 1. Next, the respective expert panelists evaluated the "isovaleric acid standard powders" in order of increasing isovaleric acid concentration, and memorized their "unpleasant odor intensities of isovaleric acid." Next, the respective expert panelists evaluated the degree of the "unpleasant odor intensity of isovaleric acid" at the time of the ingestion of a subject oral composition, and determined a powder in which the "unpleasant odor intensity of isovaleric acid" was closest to that of the composition out of the "isovaleric acid standard powders." Then, the final score of the composition was determined through discussion on the basis of the scores determined by the respective expert panelists. With regard to the scores, a smaller numerical value means that the unpleasant odor intensity of isovaleric acid is weaker.

**Table 1**

| Isovaleric acid standard powders | | |
|---|---|---|
| Score | Composition | Unpleasant odor intensity of isovaleric acid |
| 5 | 5 mass ppm of isovaleric acid | Strongly sensed |
| 4 | 0.5 mass ppm of isovaleric acid | Sensed |
| 3 | 0.1 mass ppm of isovaleric acid | Slightly sensed |
| 2 | 0.01 mass ppm of isovaleric acid | Very slightly sensed |
| 1 | 0 mass ppm of isovaleric acid | Not sensed at all |

**Table 2**

| | | | Comparative Example 1 | Comparative Example 2 | Example 1 | Example 2 | Example 3 | Example 4 | Example 5 |
|---|---|---|---|---|---|---|---|---|---|
| Composition | (A) Isovaleri c acid*¹ | mass ppm | 0.1 | 0.1 | 0.1 | 0.1 | 0.1 | 0.1 | 0.1 |
| | (B) Linalool*² | mass ppm | 0 | 0.01 | 0.1 | 1.0 | 10.0 | 100.0 | 1 000.0 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B) / (A)] | [-] | - | 0.1 | 1 | 10 | 100 | 1 000 | 10 000 |
| Sensory evaluation | Unpleasant odor intensity of isovaleric acid | | 3 | 3 | 2 | 2 | 1 | 1 | 1 |

| | | | | | | | | | |
|---|---|---|---|---|---|---|---|---|---|
| *1 Isovaleric acid: FUJIFILM Wako Pure Chemical Corporation *2 Linalool: FUJIFILM Wako Pure Chemical Corporation *3 Maltitol: Mitsubishi Corporation Life Sciences Limited | | | | | | | | | |

### Examples 6 to 10 and Comparative Examples 3 and 4

Powdered oral compositions having the compositions shown in Table 3 were obtained by the same operations as in Example 1. The respective oral compositions were subjected to analysis and sensory evaluation. The sensory evaluation was performed in the same manner as in Example 1. The results are collectively shown in Table 3.

**Table 3**

| | | | Comparative Example 3 | Comparative Example 4 | Example 6 | Example 7 | Example 8 | Example 9 | Example 10 |
|---|---|---|---|---|---|---|---|---|---|
| Composition | (A) Isovaleric acid*¹ | mass ppm | 0.5 | 0.5 | 0.5 | 0.5 | 0.5 | 0.5 | 0.5 |
| | (B) Linalool*² | mass ppm | 0 | 0.01 | 0.1 | 1.0 | 10.0 | 100.0 | 1 000.0 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B) / (A)] | [-] | - | 0.02 | 0.2 | 2 | 20 | 200 | 2 000 |
| Sensory evaluation | Unpleasant odor intensity of isovaleric acid | | 4 | 4 | 3 | 2 | 2 | 1 | 1 |

| | | | | | | | | | |
|---|---|---|---|---|---|---|---|---|---|
| *1 Isovaleric acid: FUJIFILM Wako Pure Chemical Corporation *2 Linalool: FUJIFILM Wako Pure Chemical Corporation *3 Maltitol: Mitsubishi Corporation Life Sciences Limited | | | | | | | | | |

### Examples 11 to 15 and Comparative Examples 5 and 6

Powdered oral compositions having the compositions shown in Table 4 were obtained by the same operations as in Example 1. The respective oral compositions were subjected to analysis and sensory evaluation. The sensory evaluation was performed in the same manner as in Example 1. The results are collectively shown in Table 4.

**Table 4**

| | | | Comparative Example 5 | Comparative Example 6 | Example 11 | Example 12 | Example 13 | Example 14 | Example 15 |
|---|---|---|---|---|---|---|---|---|---|
| Composition | (A) Isovaleri c acid*¹ | mass ppm | 5.0 | 5.0 | 5.0 | 5.0 | 5.0 | 5.0 | 5.0 |
| | (B) Linalool*² | mass ppm | 0 | 0.01 | 0.1 | 1.0 | 10.0 | 100.0 | 1 000.0 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.002 | 0.02 | 0.2 | 2 | 20 | 200 |
| Sensory evaluation | Unpleasant odor intensity of isovaleric acid | | 5 | 5 | 4 | 3 | 2 | 2 | 1 |

| | | | | | | | | | |
|---|---|---|---|---|---|---|---|---|---|
| *1 Isovaleric acid: FUJIFILM Wako Pure Chemical Corporation *2 Linalool: FUJIFILM Wako Pure Chemical Corporation *3 Maltitol: Mitsubishi Corporation Life Sciences Limited | | | | | | | | | |

### Examples 16 to 20 and Comparative Examples 7 and 8

Powdered oral compositions having the compositions shown in Table 5 were obtained by the same operations as in Example 1 except that geraniol was used instead of linalool. The respective oral compositions were subjected to analysis and sensory evaluation. The sensory evaluation was performed in the same manner as in Example 1. The results are collectively shown in Table 5.

**Table 5**

| | | | Comparative Example 7 | Comparative Example 8 | Example 16 | Example 17 | Example 18 | Example 19 | Example 20 |
|---|---|---|---|---|---|---|---|---|---|
| Composition | (A) Isovaleric acid*¹ | mass ppm | 0.1 | 0.1 | 0.1 | 0.1 | 0.1 | 0.1 | 0.1 |
| | (B) Geraniol*⁴ | mass ppm | 0 | 0.01 | 0.1 | 1.0 | 10.0 | 100.0 | 1 000.0 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.1 | 1 | 10 | 100 | 1 000 | 10 000 |
| Sensory evaluation | Unpleasant odor intensity of isovaleric acid | | 3 | 3 | 2 | 2 | 1 | 1 | 1 |

| | | | | | | | | | |
|---|---|---|---|---|---|---|---|---|---|
| *1 Isovaleric acid: FUJIFILM Wako Pure Chemical Corporation *3 Maltitol: Mitsubishi Corporation Life Sciences Limited *4 Geraniol: FUJIFILM Wako Pure Chemical Corporation | | | | | | | | | |

### Examples 21 to 24 and Comparative Examples 9 and 10

Powdered oral compositions having the compositions shown in Table 6 were obtained by the same operations as in Example 16. The respective oral compositions were subjected to analysis and sensory evaluation. The sensory evaluation was performed in the same manner as in Example 1. The results are collectively shown in Table 6.

**Table 6**

| | | | Comparative Example 9 | Comparative Example 10 | Example 21 | Example 22 | Example 23 | Example 24 |
|---|---|---|---|---|---|---|---|---|
| Composition | (A) Isovaleri c acid*¹ | mass ppm | 0.5 | 0.5 | 0.5 | 0.5 | 0.5 | 0.5 |
| | (B) Geraniol*⁴ | mass ppm | 0 | 0.01 | 1.0 | 10.0 | 100.0 | 1 000.0 |
| | Maltitol*³ | mass% | Balance | Balance | Balance | Balance | Balance | Balance |
| | Total | mass% | 100 | 100 | 100 | 100 | 100 | 100 |
| Calculated value | Mass ratio [(B)/(A)] | [-] | - | 0.02 | 2 | 20 | 200 | 2 000 |
| Sensory evaluation | Unpleasant odor intensity of isovaleric acid | | 4 | 4 | 3 | 2 | 2 | 1 |

| | | | | | | | | |
|---|---|---|---|---|---|---|---|---|
| *1 Isovaleric acid: FUJIFILM Wako Pure Chemical Corporation *3 Maltitol: Mitsubishi Corporation Life Sciences Limited *4 Geraniol: FUJIFILM Wako Pure Chemical Corporation | | | | | | | | |

### Examples 25 to 28 and Comparative Examples 11 and 12

Powdered oral compositions having the compositions shown in Table 7 were obtained by the same operations as in Example 16. The respective oral compositions were subjected to analysis and sensory evaluation. The sensory evaluation was performed in the same manner as in Example 1. The results are collectively shown in Table 7.

**Table 7**

| | | | Comparative Example 11 | Comparative Example 12 | Example 25 | Example 26 | Example 27 | Example 28 |
|---|---|---|---|---|---|---|---|---|
| Composition | (A) Isovaleric acid*¹ | mass ppm | 5.0 | 5.0 | 5.0 | 5.0 | 5.0 | 5.0 |
| | (B) Geraniol*⁴ | mass ppm | 0 | 0.01 | 1.0 | 10.0 | 100.0 | 1 000.0 |
| | Maltitol*³ | mass% | Balance | Balance | Balance | Balance | Balance | Balance |
| | Total | mass% | 100 | 100 | 100 | 100 | 100 | 100 |
| Calculated value | Mass ratio [(B) / (A)] | [-] | - | 0.002 | 0.2 | 2 | 20 | 200 |
| Sensory evaluation | Unpleasant odor intensity of isovaleric acid | | 5 | 5 | 4 | 3 | 2 | 1 |

| | | | | | | | | |
|---|---|---|---|---|---|---|---|---|
| *1 Isovaleric acid: FUJIFILM Wako Pure Chemical Corporation *3 Maltitol: Mitsubishi Corporation Life Sciences Limited *4 Geraniol: FUJIFILM Wako Pure Chemical Corporation | | | | | | | | |

### Examples 29 to 33 and Comparative Examples 13 and 14

Isobutyric acid and linalool were dissolved in 99.5% ethanol (manufactured by FUJIFILM Wako Pure Chemical Corporation) at the respective concentrations, and 1 g of maltitol powder (Lesys fine powder; manufactured by Mitsubishi Corporation Life Sciences Limited) and 100 uL of each of the ethanol solutions were mixed. The concentrations of the ethanol solutions were adjusted so that the final concentrations shown in Table 9 were achieved. The resultant was left to stand at 50°C for 10 minutes so that ethanol was volatilized. Thus, powdered oral compositions were prepared. The respective oral compositions were subjected to analysis and sensory evaluation. The results are collectively shown in Table 9.

### [Sensory Evaluation]

A sensory test for the "unpleasant odor intensity of isobutyric acid" of the powdered oral composition obtained in each of Examples and Comparative Examples was performed and the final score of the composition was determined in accordance with the same procedure as in the "unpleasant odor intensity of isovaleric acid" except that the concentrations of the ethanol solutions were adjusted by using an isobutyric acid reagent so that the final concentrations shown in Table 9 were achieved by one expert panelist. Isobutyric acid has an olfactory threshold value higher than that of isovaleric acid, and hence the concentration of a standard product was adjusted so that an odor almost comparable to that of isovaleric acid was achieved.

With regard to the scores, a smaller numerical value means that the unpleasant odor intensity of isobutyric acid is weaker.

**Table 8**

| Isobutyric acid standard powders | | |
|---|---|---|
| Score | Composition | Unpleasant odor intensity of isobutyric acid |
| 5 | 100 mass ppm of isobutyric acid | Strongly sensed |
| 4 | 50 mass ppm of isobutyric acid | Sensed |
| 3 | 20 mass ppm of isobutyric acid | Slightly sensed |
| 2 | 10 mass ppm of isobutyric acid | Very slightly sensed |
| 1 | 0 mass ppm of isobutyric acid | Not sensed at all |

**Table 9**

| | | | Comparative Example 13 | Comparative Example 14 | Example 29 | Example 30 | Example 31 | Example 32 | Example 33 |
|---|---|---|---|---|---|---|---|---|---|
| Composition | (A) Isobutyric acid*⁵ | mass ppm | 20 | 20 | 20 | 20 | 20 | 20 | 20 |
| | (B) Linalool*² | mass ppm | 0 | 0.01 | 0.1 | 1 | 10 | 100 | 1 000 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.0005 | 0.005 | 0.05 | 0.5 | 5 | 50 |
| Sensory evaluation | Unpleasant odor intensity of isobutyric acid | | 3 | 3 | 2 | 2 | 2 | 1 | 1 |

| | | | | | | | | | |
|---|---|---|---|---|---|---|---|---|---|
| *2 Linalool: FUJIFILM Wako Pure Chemical Corporation *3 Maltitol: Mitsubishi Corporation Life Sciences Limited *5 Isobutyric acid: FUJIFILM Wako Pure Chemical Corporation | | | | | | | | | |

### Examples 34 to 38 and Comparative Examples 15 and 16

Powdered oral compositions having the compositions shown in Table 10 were obtained by the same operations as in Example 29 except that geraniol was used instead of linalool. The respective oral compositions were subjected to analysis and sensory evaluation. The sensory evaluation was performed in the same manner as in Example 29. The results are collectively shown in Table 10.

**Table 10**

| | | | Comparative Example 15 | Comparative Example 16 | Example 34 | Example 35 | Example 36 | Example 37 | Example 38 |
|---|---|---|---|---|---|---|---|---|---|
| Composition | (A) Isobutyric acid*⁵ | mass ppm | 20 | 20 | 20 | 20 | 20 | 20 | 20 |
| | (B) Geraniol*⁴ | mass ppm | 0 | 0.01 | 0.1 | 1 | 10 | 100 | 1 000 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.0005 | 0.005 | 0.05 | 0.5 | 5 | 50 |
| Sensory evaluation | Unpleasant odor intensity of isobutyric acid | | 3 | 3 | 2 | 2 | 2 | 1 | 1 |

| | | | | | | | | | |
|---|---|---|---|---|---|---|---|---|---|
| *3 Maltitol: Mitsubishi Corporation Life Sciences Limited *4 Geraniol: FUJIFILM Wako Pure Chemical Corporation *5 Isobutyric acid: FUJIFILM Wako Pure Chemical Corporation | | | | | | | | | |

### Examples 39 to 43 and Comparative Examples 17 and 18

2-Methylbutyric acid and linalool were dissolved in 99.5% ethanol (manufactured by FUJIFILM Wako Pure Chemical Corporation) at the respective concentrations, and 1 g of maltitol powder (Lesys fine powder; manufactured by Mitsubishi Corporation Life Sciences Limited) and 100 uL of each of the ethanol solutions were mixed. The concentrations of the ethanol solutions were adjusted so that the final concentrations shown in Table 12 were achieved. The resultant was left to stand still at 50°C for 10 minutes so that ethanol was volatilized. Thus, powdered oral compositions were prepared. The respective oral compositions were subjected to analysis and sensory evaluation. The results are collectively shown in Table 12.

### [Sensory Evaluation]

A sensory test for the "unpleasant odor intensity of 2-methylbutyric acid" of the powdered oral composition obtained in each of Examples and Comparative Examples was performed and the final score of the composition was determined in accordance with the same procedure as in the "unpleasant odor intensity of isovaleric acid" except that the concentrations of the ethanol solutions were adjusted by using a 2-methylbutyric acid reagent so that the final concentrations shown in Table 11 were achieved by one expert panelist. 2-Methylbutyric acid has a olfactory threshold value higher than that of isovaleric acid, and hence the concentration of a standard product was adjusted so that an odor almost comparable to that of isovaleric acid was achieved.

With regard to the scores, a smaller numerical value means that the unpleasant odor intensity of 2-methylbutyric acid is weaker.

**Table 11**

| 2-Methylbutyric acid standard powders | | |
|---|---|---|
| Score | Composition | Unpleasant odor intensity of 2-methylbutyric acid |
| 5 | 100 mass ppm of 2-methylbutyric acid | Strongly sensed |
| 4 | 50 mass ppm of 2-methylbutyric acid | Sensed |
| 3 | 20 mass ppm of 2-methylbutyric acid | Slightly sensed |
| 2 | 10 mass ppm of 2-methylbutyric acid | Very slightly sensed |
| 1 | 0 mass ppm of 2-methylbutyric acid | Not sensed at all |

**Table 12**

| | | | Comparative Example 17 | Comparative Example 18 | Example 39 | Example 40 | Example 41 | Example 42 | Example 43 |
|---|---|---|---|---|---|---|---|---|---|
| Composition | (A) 2-Methylbutyric acid*⁶ | mass ppm | 20 | 20 | 20 | 20 | 20 | 20 | 20 |
| | (B) Linalool*² | mass ppm | 0 | 0.01 | 0.1 | 1 | 10 | 100 | 1 000 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.0005 | 0.005 | 0.05 | 0.5 | 5 | 50 |
| Sensory evaluation | Unpleasant odor intensity of 2-methylbutyric acid | | 3 | 3 | 2 | 2 | 2 | 1 | 1 |

| | | | | | | | | | |
|---|---|---|---|---|---|---|---|---|---|
| *2 Linalool: FUJIFILM Wako Pure Chemical Corporation *3 Maltitol: Mitsubishi Corporation Life Sciences Limited *6 2-Methylbutyric acid: FUJIFILM Wako Pure Chemical Corporation | | | | | | | | | |

### Examples 44 to 48 and Comparative Examples 19 and 20

Powdered oral compositions having the compositions shown in Table 13 were obtained by the same operations as in Example 39 except that geraniol was used instead of linalool. The respective oral compositions were subjected to analysis and sensory evaluation. The sensory evaluation was performed in the same manner as in Example 39. The results are collectively shown in Table 13.

**Table 13**

| | | | Comparative Example 19 | Comparative Example 20 | Example 44 | Example 45 | Example 46 | Example 47 | Example 48 |
|---|---|---|---|---|---|---|---|---|---|
| Composition | (A) 2-Methylbutyric acid*⁶ | mass ppm | 20 | 20 | 20 | 20 | 20 | 20 | 20 |
| | (B) Geraniol*⁴ | mass ppm | 0 | 0.01 | 0.1 | 1 | 10 | 100 | 1 000 |
| | Maltitol*³ | mass% | Balance | Balance | Balance | Balance | Balance | Balance | Balance |
| | Total | mass% | 100 | 100 | 100 | 100 | 100 | 100 | 100 |
| Calculated value | Mass ratio [(B)/(A)] | [-] | - | 0.0005 | 0.005 | 0.05 | 0.5 | 5 | 50 |
| Sensory evaluation | Unpleasant odor intensity of 2-methylbutyric acid | | 3 | 3 | 2 | 2 | 2 | 1 | 1 |

| | | | | | | | | | |
|---|---|---|---|---|---|---|---|---|---|
| *3 Maltitol: Mitsubishi Corporation Life Sciences Limited *4 Geraniol: FUJIFILM Wako Pure Chemical Corporation *6 2-Methylbutyric acid: FUJIFILM Wako Pure Chemical Corporation | | | | | | | | | |

It is found from Table 2 to Table 13 that, when linalool and/or geraniol is incorporated at a predetermined or larger amount, an oral composition suppressed in unpleasant odor of each of isovaleric acid, isobutyric acid, and 2-methylbutyric acid is obtained.

## Claims

1. An oral composition, comprising the following components (A) and (B):
(A) 0.01 mass ppm or more of a branched fatty acid having 6 or less carbon atoms; and
(B) 0.05 mass ppm or more of at least one monoterpene alcohol selected from the group consisting of linalool and geraniol.

2. The oral composition according to claim 1, wherein a mass ratio of the component (B) to the component (A), [(B)/(A)], is from 0.003 to 20 000.

3. The oral composition according to claim 1 or 2, wherein a content of the component (A) is from 0.01 mass ppm to 30 mass ppm.

4. The oral composition according to any one of claims 1 to 3, wherein a content of the component (B) is from 0.05 mass ppm to 3 000 mass ppm.

5. The oral composition according to any one of claims 1 to **4,** wherein the component (A) is one or more selected from the group consisting of isobutyric acid, 2-methylbutyric acid, and isovaleric acid.

6. An unpleasant odor suppressant for a branched fatty acid having 6 or less carbon atoms, the unpleasant odor suppressant comprising at least one monoterpene alcohol selected from the group consisting of linalool and geraniol as an active ingredient.

7. A method of suppressing an unpleasant odor of a branched fatty acid having 6 or less carbon atoms, the method comprising causing at least one monoterpene alcohol selected from the group consisting of linalool and geraniol to coexist with respect to the branched fatty acid having 6 or less carbon atoms.

8. The unpleasant odor suppressant according to claim 6 or the method of suppressing an unpleasant odor according to claim **7,** wherein the branched fatty acid having 6 or less carbon atoms is one or more selected from the group consisting of isobutyric acid, 2-methylbutyric acid, and isovaleric acid.
